(19) Europäisches Patentamt
European Patent Office
Office européen des brevets

(11) Veröffentlichungsnummer: **0 003 284**
**B1**

(12) # EUROPÄISCHE PATENTSCHRIFT

(21) Anmeldenummer: **79100057.3**

(22) Anmeldetag: **10.01.79**

(51) Int. Cl.³: **C 07 D 403/04,**
A 01 N 43/64,//
C 07 D 253/08

(54) **Fungizide und bakterizide Mittel auf Basis von 3-Azolylbenzotriazinen und -benzotriazin-1-oxiden und deren Verwendung zur Bekämpfung von Pflanzenkrankheiten**

(30) Priorität: **20.01.78 DE 2802488**

(43) Veröffentlichungstag der Anmeldung:
**08.08.79 Patentblatt 79/16**

(45) Bekanntmachung des Hinweises auf die Patenterteilung:
**07.01.81 Patentblatt 81/01**

(84) Benannte Vertragsstaaten:
**BE CH DE FR GB IT NL SE**

(56) Entgegenhaltungen:
**DE-A-2 306 512**
**US-A-2 489 355**

(73) Patentinhaber: **BAYER AG**
**Zentralbereich Patente, Marken und Lizenzen**
**D-5090 Leverkusen 1, Bayerwerk (DE)**

(72) Erfinder: **Sasse, Klaus, Dr.**
**Pützweg 13**
**D-5060 Berg.-Gladbach (DE)**
**Gauss, Walter, Dr.**
**Ludwig-Aschoff-Strasse 4**
**D-5000 Köln 80 (DE)**
**Frohberger, Paul-Ernst, Dr.**
**Willi-Baumeister-Strasse 5**
**D-5090 Leverkusen (DE)**
**Kraus, Peter, Dr.**
**Düsseldorfer Strasse 43**
**D-5000 Köln 80 (DE)**
**Paul, Volker, Dr.**
**Ahornstrasse 5**
**D-5650 Solingen (DE)**

**EP 0 003 284 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Fungizide und bakterizide Mittel auf Basis von 3-Azolylbenzotriazinen und -benzotriazin-1-oxiden und deren Verwendung zur Bekämpfung von Pflanzenkrankheiten

Die vorliegende Erfindung betrifft die Verwendung von 3-Azolyl-benzotriazinen und -benzotriazin-1-oxiden als fungizide und bakterizide Mittel zur Bekämpfung von Pflanzenkrankheiten.

Es ist bereits bekanntgeworden, daß Benzo-1,2,4-triazin-1-oxide mit Halogen-, Amino-, Hydrazino-, Alkoxy- und Alkylmercapto-Substituenten in 3-Stellung fungizide, herbizide und akarizide Eigenschaften aufweisen (vgl. DL—PS 83 869). Fungizide Eigenschaften wurden jedoch (in vitro) nur bei Verbindungen mit Chlorsubstituenten in 3-Stellung aufgefunden. Praktische Bedeutung haben diese Stoffe nicht erlangt. Weiterhin ist bekannt (vgl. DE—OS 25 38 179), daß 3-Alkoxy-benzo-1,2,4-triazine mit zusätzlichen Substituenten in 7-Stellung fungizid und bakterizid wirken. Deren Wirkungsspektrum ist jedoch relativ begrenzt. Der Schwerpunkt ihrer Wirkung liegt bei Helminthosporium-Arten und Rosterkrankungen. Die bakterizide Wirkung gegen Xanthomonas-Arten ist für einen praktischen Einsatz nicht ausreichend.

Weiterhin sind als Stoffe die 3-Imidazolyl-benzo-1,2,4-triazine und deren 1-Oxide bekannt (vgl. DE—OS 23 06 512), auch ist bekannt, daß die erstgenannte Verbindungsgruppe eine entzündungshemmende Wirkung besitzt und daher als Arzneimittel Verwendung finden kann.

Es wurde nun gefunden, daß die 3-Azolyl-benzo-1,2,4-triazine und -benzo-1,2,4-triazin-1-oxide der allgemeinen Formel

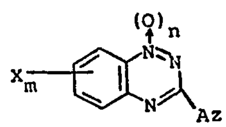

(I)

in welcher

X für Halogen, Alkyl-, Alkoxy-, Alkylmercapto- oder Alkylsulfonyl-Gruppen mit jeweils bis zu 4 Kohlenstoffatomen, oder für eine Nitro- oder Trifluormethyl-Gruppe steht,

m für ganze Zahlen von 0 bis 4 steht,

n für die Zahlen 0 oder 1 steht und

Az für einen über Stickstoff gebundenen fünfgliedrigen heteroaromatischen Ring mit 2 oder 3 Stickstoffatomen steht, der gegebenenfalls substituiert sein kann durch Alkyl, Alkoxy oder Alkylmercapto mit jeweils 1 bis 6 Kohlenstoffatomen, durch Halogen, Hydroxy, durch Aryl- und Aralkyl-Reste, ferner durch Amino-, Mono- oder Dialkylamino-Gruppen mit 1 bis 2 Kohlenstoffatomen in den jeweiligen Alkylgruppen, durch Alkoxyalkoxy-, Acylamino-, Carboxyl-Carbonsäureester- und gegebenenfalls N-substituierte Carbonamido-Gruppen und durch die Cyano-Gruppe,

starke fungizide und bakterizide Eigenschaften aufweisen.

Die erfindungsgemäß verwendbaren Verbindungen der allgemeinen Formel I werden erhalten, wenn man

a) 3-substituierte Benzo-1,2,4-triazin (-1-oxid)e der Formel

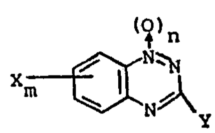

(II)

in welcher

x, m und n die oben genannte Bedeutung besitzen und

Y für Halogen, eine Hydroxy- oder Sulfogruppe steht,

mit einer gegebenenfalls substituierten fünfgliedrigen heteroaromatischen Ringverbindung (einem Azol) der allgemeinen Formel

H—Az (III)

in welcher

Az die weiter oben angegebene Bedeutung besitzt, und das angegebene H-Atom mit einem N-Atom des fünfgliedrigen heterocyclischen Ringsystems verknüpft ist,

umsetzt, und gegebenenfalls die dabei erhaltenen Reaktionsprodukte der nachfolgenden Reduktion in 1-Stellung unterwirft, oder

b) im Falle der Herstellung von Pyrazolyl-Verbindungen (Az steht in Formel I dann für einen gegebenenfalls substituierten Pyrazolyl-Rest) 3-Hydrazinobenzo-1,2,4-triazin(1-oxid)e der Formel

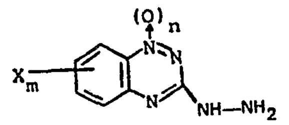

(IV)

in welcher
X, m und n die oben angegebene Bedeutung besitzen,

mit Dicarbonyl-Verbindungen bzw. $\beta$-Oxocarbonsäure-Derivaten oder solchen Verbindungen umsetzt, die während des Reaktionsverlaufs 1,3-Dicarbonyl-Verbindungen bzw. $\beta$-Oxocarbonsäuren freizusetzen vermögen, und die Reaktionsprodukte gegebenenfalls der nachfolgenden Reduktion in 1-Stellung unterwirft.

Gegenüber den oben genannten verwandten Stoffen besitzen die erfindungsgemäß verwendbaren Verbindungen ein deutlich breiteres fungizides und bakterizides Wirkungspektrum gegen pflanzenpathogene Krankheitserreger. Besonders hervorzuheben sind ihre hervorragenden bakteriziden Eigenschaften, z.B. gegenüber Xanthomonas-Arten, zu deren Bekämpfung voll befriedigende Mittel bisher nicht zur Verfügung standen. Die erfindungsgemäßen Wirkstoffe stellen somit eine wertvolle Bereicherung der Technik dar.

Verwendet man zur Herstellung der erfindungsgemäß verwendbaren Verbindungen entsprechend der Verfahrensvariante a) 3-Chlor-benzo-1,2,4-triazin-1-oxid und Imidazol bzw. Benzo-1,2,4-triazin-1-oxid-3-sulfonsäure und 1,2,4-Triazol als Ausgangsstoffe, so kann der Reaktionsablauf durch die folgenden Reaktionsgleichungen wiedergegeben werden:

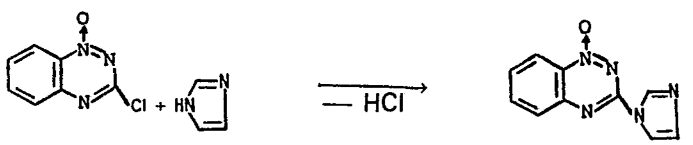


bzw.

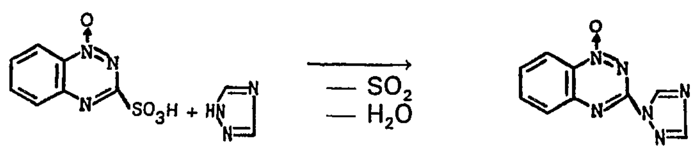


Verwendet man entsprechend der Verfahrensvariante b) 7-Chlor-3-hydrazino-benzotriazin-1-oxid und Acetylaceton bzw. 1,1,3,3-Tetramethoxy-propan als Reaktionspartner, so nimmt die Reaktion folgenden Verlauf:

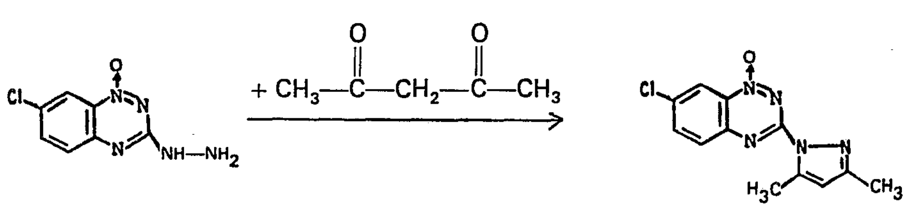

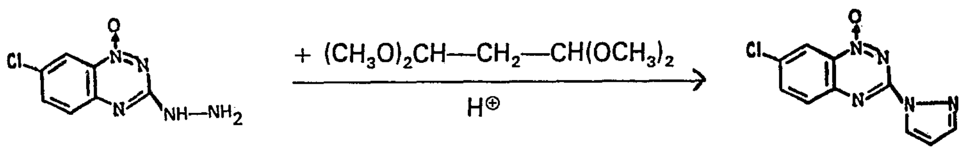


Führt man in Anschluß an die Verfahren a) oder b) nach eine Reduktion der N-Oxide durch, so kann eine solche Reaktion wie folgt dargestellt werden, wobei als Beispiel die Reaktion von 7-Chlor-3-pyrazolyl-benzo-1,2,4-triazin-1-oxid mit katalytisch erregtem Wasserstoff skizziert sei:

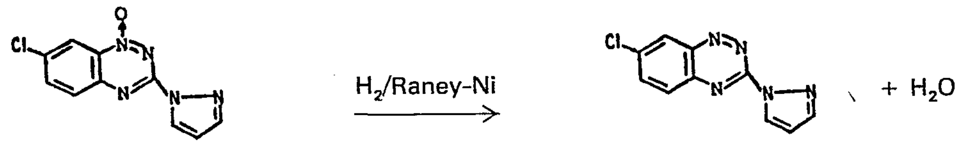


Mann kann zu den (in 1-Stellung) saurestoff-freien 3-Pyrazolyl-(1)-benzotriazinen nach Verfahrensvariante b) aber auch dadurch gelangen, daß man anstelle der 3-Hydrazino-benzotriazin-1-oxide die 3-Hydrazino-benzotriazine (in Formel IV steht n für die Zahl 0) mit 1,3-Dicarbonyl-Verbindungen bzw. deren Derivaten umsetzt. Mit 3-Hydrazino-benzotriazin und Acetylaceton als Reaktionspartnern nimmt die Reaktion z.B. folgenden Verlauf:

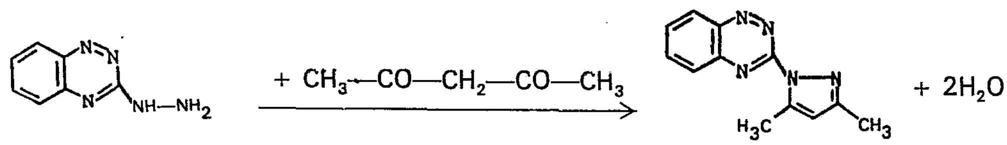


Von praktischem Interesse sind wegen ihrer guten Zugänglichkeit insbesondere diejenigen erfindungsgemäß verwendbaren Verbindungen der allgemeinen Formel I, in denen der Index *n* den Wert 1 annimmt.

Die für die Reaktion nach Verfahren a) als Vorprodukte benötigten in 3-Stellung substituierten Benzo-1,2,4-triazine bzw. Benzo-1,2,4-triazin-1-oxide sind durch die allgemeine Formel II definiert. In dieser Formel steht X vorzugsweise für Chlor oder Brom, ferner vorzugsweise für Methyl oder Trifluormethyl, für Methoxy oder Methylmercapto, und endlich vorzugsweise für die Nitro-Gruppe. Der Index m steht vorzugsweise für eine ganze Zahl von 0 bis 3 und der Index n hat vorzugsweise die bei der Erfindungsdefinition genannten Bedeutungen. Der Rest Y steht vorzugsweise für Chlor, Brom, die Hydroxy- oder die Sulfo-Gruppe ($SO_3H$). Die Verbindungen der Formel II sind prinzipiell bekannt. Man gewinnt sie beispielsweise durch Einwirkung von Phosphoroxyhalogeniden auf 3-Hydroxy-benzotriazin-1-oxide (siehe z.B. J.Chem.Soc. *1957*, 3186; J.Org.Chem. *24*, 813 (1959)). Soweit entsprechend im Benzolkern substituierte 3-Chlor-benzotriazin-1-oxide bisher noch nicht vorbeschrieben sind, können diese auf völlig analogem Wege dargestellt werden. Benzotriazin-1-oxid-3-sulfonsäuren können, wie ebenfalls grundsätzlich beschrieben, durch Oxidation von 3-Mercapto-benzotriazin-1-oxiden entsprechend den Literaturangabaen hergestellt werden.

Die weiterhin als Reaktionspartner für das Verfahren a) benötigten Azole sind durch die allgemeine Formel III dargestellt. Bei diesen fünfgliedrigen heteroaromatischen Verbindungen mit 2 oder 3 Ringstickstoffatomen steht Az vorzugsweise für die folgenden Gruppen:

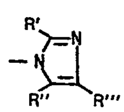
Imidazolyl-(1)

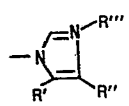
Pyrazolyl-(1)

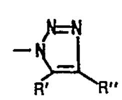
1,2,3-Triazolyl-(1)

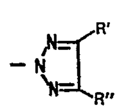
1,2,3-Triazolyl-(2)

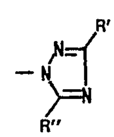
1,2,4-Triazolyl-(1)

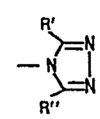
1,2,4-Triazolyl-(4)

Die dargestellten Azolyl-Reste können substituiert sein:

R', R'', R''' stehen für Wasserstoff, Alkyl, Alkoxy oder Alkylmercapto mit jeweils 1 bis 6 Kohlenstoffatomen, für Halogen, Hydroxy, für Aryl- und Aralkyl-Reste, ferner für Amino-, Mono- oder Dialkylamino-Gruppen mit 1 bis 2 Kohlenstoffatomen in den jeweiligen Alkylgruppen, für Alkoxyalkoxy-, Acylamino-, Carboxyl-, Carbonsäureester- und gegebenenfalls N-substituierte Carbonamido-Gruppen und für die Cyano-Gruppe.

Vorzugsweise stehen die Reste R', R'' und R''' für Wasserstoff, Alkyl- oder Alkoxy-Gruppen mit 1 bis 5 Kohlenstoffatomen, für Methylmercapto, für Chlor, Brom, ferner für Phenyl und Benzyl, für Amino, Methylamino und Dimethylamino, weiterhin vorzugsweise für Methoxy- und Äthoxycarbonyl, für die Acetylamino- und die Cyano-Gruppe. Als Beispiele für Verbindungen der Formel III sind zu nennen:

Pyrazol
3-Methyl-pyrazol
4-Methyl-pyrazol
3-Aethyl-pyrazol
4-Aethyl-pyrazol
4-Propyl-pyrazol
4-Isopropyl-pyrazol
4-Butyl-pyrazol
4-Pentyl-pyrazol
4-Phenyl-pyrazol
4-Benzyl-pyrazol
3,4-Dimethyl-pyrazol
3,5-Dimethyl-pyrazol
3,4-Diäthyl-pyrazol
3,5-Diäthyl-pyrazol
4-Chlor-pyrazol
4-Brom-pyrazol
3,5-Dimethyl-4-chlor-pyrazol
3,5-Dimethyl-4-brom-pyrazol
4-Methoxy-pyrazol
4-Aethoxy-pyrazol
4-Propoxy-pyrazol
4-Isopropoxy-pyrazol
4-Methoxyäthoxy-pyrazol
Imidazol
2-Methyl-imidazol
2-Butyl-imidazol
2,4,5-Trimethyl-imidazol
2-Methoxy-imidazol
2-Methylmercapto-imidazol
4,5-Dichlor-imidazol
2-Acetamino-imidazol
4,5-Dichlor-imidazol-2-carbonsäuremethylester
4,5-Dichlor-imidazol-2-carbonsäure-dimethylamid
4,5-Dichlor-2-cyan-imidazol
1,2,3-Triazol
4,5-Dimethyl-1,2,3-triazol
4-Phenyl-1,2,3-triazol
4,5-Diphenyl-1,2,3-triazol
1,2,3-Triazol-4-carbonsäure-äthylester
1,2,3-Triazol-4,5-dicarbonsäure-diäthylester
1,2,4-Triazol
3-Methyl-1,2,4-triazol
3-Methylmercapto-1,2,4-triazol
3-Amino-1,2,4-triazol

Die für die Reaktion nach Variante b) als Vorprodukte benötigten 3-Hydrazino-benzo-1,2,4-triazine bzw. 3-Hydrazino-benzo-1,2,4-triazin-1-oxide sind durch die allgemeine Formel IV definiert. In dieser Formel haben X, m und n die oben bei Besprechung der Formel II angegebenen vorzugsweisen Bedeutungen.

Die durch die Formel IV wiedergegebenen 3-Hydrazino-benzotriazin-1-oxide sind grundsätzlich bekannt (siehe z.B. J.Chem.Soc. *1957*, 3186 und J.Org.Chem. *24*, 813 (1959)). Soweit diese mit der gewünschten Substitution im Benzolkern noch nicht vorbeschrieben sind, lassen sie sich nach bekannten Verfahren, z.B. durch Umsetzung der 3-Chlor-benzotriazin-1-oxide mit Hydrazin. Bekannt ist weiterhin das in 1-Stellung nicht oxydierte 3-Hydrazino-benzo-1,2,4-triazin (vgl. die beiden oben genannten Publikationen). Die noch nicht beschriebenen 3-Hydrazino-benzotriazine mit Substituenten im Benzolkern lassen sich völlig analog dadurch herstellen, indem man 3-Hydrazino-benzotriazin-1-oxide der Reduktion unterwirft. Die Reduktionsbedingungen sind im großen und ganzen die gleichen wie dies noch für die entsprechende Reduktion der 3-Azolylbenzo-1,2,4-triazin-1-oxide (Formel I, n steht für 1) beschrieben wird. Besonders geeigne sind z.B. Hydrogensulfide als Reduktionsmittel.

Die weiterhin als Reaktionspartner für das Verfahren b) benötigten 1,3-Dicarbonyl-Verbindungen bzw. ß-Oxocarbonsäure-Derivate sind grundsätzlich bekannte Stoffe. Als solche kommen in Betracht:
Malon -dialdehyd (Propan-dion-1,3)
Chlormalondialdehyd
Brommalondialdehyd

2-Methyl-propan-dion-1,3
Butan-dion-1,3
Pentan-dion-1,3
Pentan-dion-2,4 (Acetylaceton)
Heptan-dion-3,5
3-Dimethylamino-acrolein
2-Methyl-3-dimethylamino-acrolein
2-Aethyl-3-dimethylamino-acrolein
2-Propyl-3-dimethylamino-acrolein
2-Methoxy-3-dimethylamino-acrolein
2-Aethoxy-3-dimethylamino-acrolein
2-Propoxy-3-dimethylamino-acrolein
2-Isopropoxy-3-dimethylamino-acrolein,
1,1,3,3-Tetramethoxy-propan
4,4-Dimethoxy-butanon-2 (Acetoacetaldehyd-dimethyl-acetal)
Formylessigsäureäthylester
Acetessigsäure-methylester
Acetessigsäure-äthylester
Diketen
Cyanaceton
Cyanacetophenon
$\alpha$-Cyan-phenylacetaldehyd
Aethoxymethylencyanessigsäure-äthylester
Aethoxymethylenmaloninitril

Arbeitet man gemäß Verfahrensvariante a), so wird man die Umsetzung in einem geeigneten Verdünnungsmittel durchführen. Als solche kommen inerte Lösungsmittel infrage, wie z.B. aliphatische oder aromatische Kohlenwasserstoffe, Halogenkohlenwasserstoffe, Äther (wie Diäthyläther, Tetrahydrofuran, Dioxan, Glykoldimethyläther), Ester (Essigsäureäthylester), Ketone (Aceton, Methylisobutylketon), Dimethylformamid, Dimethylsulfoxid oder auch deren Gemische mit Wasser.

Weiterhin werden bei Durchführung des Verfahrens a) als Hilfsstoffe Säurebindemittel benötigt, wenn solche Verbindungen der Formel II zur Verwendung gelangen, in denen Y für Halogen oder für die $SO_3H$-Gruppe steht. Zur Bindung der bei der Umsetzung freiwerdenden Säure ist die Zugabe der mindestens äquimolaren Menge einer Base zweckmäßig. Als solche können verwendet werden: Erdalkalioxide und -hydroxide, Alkali- und Erdalkalicarbonate und -acetate, tertiäre Amine oder heterocyclische Stickstoffbasen oder aber auch ein zweites Mol des als Reaktionskomponente eingesetzten Azols. Man kann jedoch auch so verfahren, daß man das als säurebindendes Mittel eingesetzte tertiäre Amin (z.B. Pyridin) im Überschuß und damit gleichzeitig als Lösungsmittel einsetzt. Weiterhin können unter Einsparung eines gesonderten säurebindenden Mittels auch Metallderivate der Azole, z.B. deren Alkaliverbindungen, Halogenmagnesium-Derivate oder aber auch ihre N-Trialkylsilyl-Derivate eingesetzt werden.

Im Falle des Einsatzes von solchen Verbindungen der Formel II, in denen Y eine OH-Gruppe bedeutet, ist eine wasserbindende Substanz als Kondensationsmittel erforderlich. Hierfür hat sich insbesondere Phosphoroxychlorid als brauchbar erwiesen, das in mindestens äquimolarer Menge aufgewendet werden muß, wobei inerte Lösungsmittel, z.B. aliphatische und aromatische (Chlor-)Kohlenwasserstoffe, als Verdünnungsmittel dienen können. Zweckmäßig ist aber der Einsatz von Phosphoroxychlorid im Überschuß, und zwar in solcher Menge, daß das Reaktionsgemisch in flüssiger Form rührbar bleibt. Die Reaktionstemperaturen können beim Verfahren a) in einem größeren Bereich variiert werden. Im allgemeinen arbeitet man zwischen etwa 0 und etwa 130°C, vorzugsweise bei 15 bis 100°C.

Bei der Umsetzung gemäß Verfahren a) von Benzo-1,2,4-triazin-1-oxiden der Formel II (wobei n für die Zahl 1 steht) mit Azolen der Formel III fallen Reaktionsprodukte der Formel I an, die in 1-Stellung die N-Oxid-Gruppierung enthalten. Um diese gegebenenfalls in Verbindungen der Formel I mit nichtoxydierter 1-Stellung überzuführen, ist eine nachfolgende Reduktion erforderlich. Hierfür sind verschiedene Reduktionsmittel geeignet, z.B. nascierender Wasserstoff (aus Zink, Zinn oder Eisen und Säure) oder katalytisch erregter Wasserstoff mit z.B. Raney-Nickel als Katalysator oder schwefelhaltige Säuren niederer Werktigkeitsstufen bzw. deren Salze, z.B. Sulfide, Dithionite, Sulfite etc. Diese Reduktionsreaktionen führt man vorzugsweise in Alkoholen, Tetrahydrofuran, Dioxan oder Dimethylformamid durch, gegebenenfalls im Gemisch mit Wasser. Die Reaktionstemperaturen betragen 0 bis 100°C, vorzugsweise 10 bis 70°C.

Bei der Durchführung des Verfahrens a) setzt man die Verbindungen der Formel II und die Azole der Formel III vorzugsweise in äquimolaren Mengen ein. Unter- oder Überschreitungen um bis zu etwa 20% können jedoch ohne wesentliche Veränderung der Ausbeute in Kauf genommen werden. Die Aufarbeitung kann zweckmäßigerweise so vorgenommen werden, daß man den Hauptteil absdestilliert, den Rückstand mit Wasser verdünnt, bei Anwesenheit von Basen stark verdünnte Salzsäure hinzufügt, das

angefallene Produkt absaugt und trocknet. Zur Reinigung kann das Rohprodukt aus einem geeigneten Lösungsmittel umkristallisiert werden.

Wie schon erwähnt, kann man zur Herstellung solcher erfindungsgemäß verwendbarer Verbindungen der Formel I bei denen Az den Rest eines Pyrazols bedeutet, auch nach Verfahren b) arbeiten. Hierbei bringt man ein 3-Hydrazino-benzo-1,2,4-triazin oder -1,2,4-triazin-1-oxid der Formel IV mit einer 1,3-Dicarbonyl-Verbindung oder einem ß-Oxocarbonsäure-Derivat zur Umsetzung; an Stelle der letzteren Verbindung können auch solche Stoffe Verwendung finden, die 1,3-Dicarbonyl-Verbindungen bzw. ß-Oxocarbonsäuren in Freiheit zu setzen vermögen.

Die Umsetzung gemäß Verfahren b) läßt sich in allen gängigen Verdünnungsmitteln durchführten, die nicht selbst mit Hydrazin- oder Carbonyl-Verbindungen in Reaktion treten. Als solche sind z.B. geeignet: aliphatische und aromatische (Halogen-)Kohlenwasserstoffe, Äther wie Diäthylether, Tetrahydrofuran oder Dioxan, Alkohole, Dimethylformamid, Dimethylsulfoxid und dergleichen, sowie auch Wasser oder Gemische aus den vorgenannten Lösungsmitteln mit Wasser.

Beim Verfahren b) sind oft Hilfsstoffe notwendig: Werden nämlich Acetale bzw. Ketale der 1,3-Dicarbonyl-Verbindungen eingesetzt, so ist in der Regel der Zusatz von Carbonsäuren oder Mineralsäuren, z.B. Salz-, Schwefel- oder Essigsäure, erforderlich, um hieraus die Carbonylfunktionen in Freihet zu setzten. Hierfür genügen oft katalytische Mengen dieser Mineralsäuren von z.B. 1 Mol-%, jedoch kann es in einzelnen Fällen auch zwäckmäßig sein, diese in äquimolaren Mengen anzuwenden.

Die Reaktionstemperaturen können beim Verfahren b) in einem größeren Bereich variiert werden. Im allgemeinen arbeitet man zwischen etwa 0 und 100°C, vorzugsweise zwischen 10 und 80°C.

Bei der Durchführung des Verfahrens b) setzt man die Hydrazino-Verbindungen der Formel IV und die 1,3-Dicarbonyl-Verbindungen vorzugsweise im äquimolaren Verhältnis ein, jedoch ist es auch möglich und für die Abtrennung der Reaktionsprodukte unschädlich, wenn die 1,3-Dicarbonyl-Verbindung im Überschuß (z.B. bis zu einem weiteren Mol) angewendet wird. Die Aufarbeitung des Reaktionsgemisches gestaltet sich dadurch einfach, daß das Reaktionsprodukt meistens nach dem Abkühlen ausfällt und ohne weitere Operationen abgesaugt werden kann.

Als erfindungsgemäß verwendbare Verbindungen der Formel I seien im einzelnen genannt:

3-Pyrazolyl-(1)-benzotriazin-1-oxid
3-Pyrazolyl-(1)-benzotriazin
3-Imidazolyl-(1)-benzotriazin-1-oxid
3-Imidazolyl-(1)-benzotriazin
3-Triazolyl-(1)-benzotriazin-1-oxid
3-Triazolyl-(1)-benzotriazin
3-Pyrazolyl-(1)-6-chlor-benzotriazin
3-Imidazolyl-(1)-6-chlor-benzotriazin
3-Imidazolyl-(1)-6-chlor-benzotriazin-1-oxid
3-Pyrazolyl-(1)-7-chlor-benzotriazin-1-oxid
3-Pyrazolyl-(1)-7-chlor-benzotriazin
3-(3-Methyl-pyrazolyl-(1))-7-chlor-benzotriazin
3-(3,5-Dimethyl-pyrazolyl-(1))-7-chlor-benzotriazin
3-(3,4,5-Trimethyl-pyrazolyl-(1))-7-chlor-benzotriazin-1-oxid
3-(4-Aethyl-pyrazolyl-(1))-7-chlor-benzotriazin-1-oxid
3-(4-Chlor-pyrazolyl-(1))-7-chlor-benzotriazin-1-oxid
3-(4-Methoxy-pyrazolyl-(1))-7-chlor-benzotriazin-1-oxid
3-(4-Isopropoxy-pyrazolyl-(1))-7-chlor-benzotriazin-1-oxid
3-(5-Amino-4-carbäthoxy-pyrazolyl-(1))-7-chlor-benzotriazin-1-oxid
3-(5-Hydroxy-pyrazolyl-(1))-7-chlorbenzotriazin
3-(3-Methyl-5-hydroxy-pyrazolyl-(1))-7-chlor-benzotriazin-1-oxid
3-(3-Methyl-5-hydroxy-pyrazolyl-(1))-7-chlor-benzotriazin
3-Imidazolyl-(1)-7-chlor-benzotriazin-1-oxid
3-Imidazolyl-(1)-7-chlor-benzotriazin
3-(2-Methyl-imidazolyl-(1))-7-chlor-benzotriazin
3-(2,4,5-Trimethyl-imidazolyl-(1))-7-chlor-benzotriazin
3-(2-Methoxy-imidazolyl-(1))-7-chlor-benzotriazin
3-(2-Methylmercapto-imidazolyl-(1))-7-chlor-benzotriazin
3-(4,5-Dichlor-imidazolyl-(1))-7-chlor-benzotriazin
3-(2-Acetamino-imidazolyl-(1))-7-chlor-benzotriazin
3-(4,5-Dichlor-2-cyan-imidazolyl-(1))-7-chlor-benzotriazin
3-(1,2,3-Triazolyl-(1)-7-chlor-benzotriazin-1-oxid
3-(1,2,3-Triazolyl-(1))-7-chlor-benzotriazin
3-(1,2,3-Triazolyl-(2))-7-chlor-benzotriazin-1-oxid
3-(1,2,3-Triazolyl-(2))-7-chlor-benzotriazin
3-(1,2,4-Triazolyl-(1))-7-chlor-benzotriazin-1-oxid
3-(1,2,4-Triazolyl-(1))-7-chlor-benzotriazin

3-(3-Methyl-1,2,4-triazolyl-(1))-7-chlor-benzotriazin
3-(3-Methylmercapto-1,2,4-triazolyl-(1))-7-chlor-benzotriazin
3-(3-Amino-1,2,4-triazolyl-(1))-7-chlor-benzotriazin
3-Pyrazolyl-(1)-5,7-dichlor-benzotriazin
3-(3,5-Dimethyl-pyrazolyl-(1))-5,7-dichlor-benzotriazin-1-oxid
3-(4-Aethyl-pyrazolyl-(1))-5,7-dichlor-benzotriazin
3-(4-Chlor-pyrazolyl-(1))-5,7-dichlor-benzotriazin
3-(4-Methoxy-pyrazolyl-(1))-5,7-dichlor-benzotriazin
3-Imidazolyl-(1)-5,7-dichlor-benzotriazin-1-oxid
3-Imidazolyl-(1)-5,7-dichlor-benzotriazin
3-Triazolyl-(1)-5,7-dichlor-benzotriazin-1-oxid
3-Triazolyl-(1)-5,7-dichlor-benzotriazin
3-Pyrazolyl-(1)-6,7-dichlor-benzotriazin
3-Imidazolyl-(1)-6,7-dichlor-benzotriazin
3-Pyrazolyl-(1)-5,7,8-trichlor-benzotriazin
3-Imidazolyl-(1)-5,7,8-trichlor-benzotriazin
3-Pyrazolyl-(1)-7-brom-benzotriazin
3-Imidazolyl-(1)-7-brom-benzotriazin
3-Pyrazolyl-(1)-7-methyl-benzotriazin
3-Imidazolyl-(1)-7-methyl-benzotriazin
3-Pyrazolyl-(1)-7-butyl-benzotriazin
3-Pyrazolyl-(1)-7-trifluormethyl-benzotriazin
3-Imidazolyl-(1)-7-trifluormethyl-benzotriazin-1-oxid
3-Imidazolyl-(1)-7-trifluormethyl-benzotriazin
3-Pyrazolyl-(1)-7-methoxy-benzotriazin
3-Pyrazolyl-(1)-7-butoxy-benzotriazin
3-Imidazolyl-(1)-7-methylmercapto-benzotriazin
3-Imidazolyl-(1)-7-butylmercapto-benzotriazin
3-Imidazolyl-(1)-7-methylsulfonyl-benzotriazin
3-Imidazolyl-(1)-7-nitro-benzotriazin-1-oxid
3-Triazolyl-(1)-7-nitro-benzotriazin-1-oxid

Die Wirkstoffe weisen eine starke fungizide und eine bakterizide Wirkung auf. Sie schädigen Kulturpflanzen in den zur Bekämpfung von Pilzen und Bakterien notwendigen Konzentrationen nicht. Aus diesem Grunde sind sie für den Gebrauch als Pflanzenschutzmittel zur Bekämpfung von Pilzen und Bakterien geeignet. Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz eingesetzt zur Bekämpfung von Bakterien aus der Familie der Pseudomonadaceae, z.B. von Pseudomonas solanacearum, Pseudomonas lachrymans, Pseudomonas syringae, Xanthomonas citri, Xanthomonas oryzae und Xanthomonas vesicatoria, der Familie der Enterobacteriaceae, z.B. von Erwinia amylovora, bzw. der Familie der Corynebacteriaceae, ferner aus der Familie der Rhizobiaceae, z.B. von Agrobacterium tumefaciens.

Die Wirkstoffe können zur Behandlung oberirdischer Pflanzenteile, zur Saatgutbehandlung und zur Bodenbehandlung Verwendung finden.

Die Wirkstoffe weisen einen hohen Wirkungsgrad und eine große Wirkungsbreite auf. Sie sind einfach zu handhaben und können zur Bekämpfung unerwünschten Pilzund Bakterienwachstums praktisch eingesetzt werden.

Ihre gute Pflanzenverträglichkeit erlaubt eine Anwendung gegen pilzliche Planzenkrankheiten durch Behandlung der stehenden Kulturpflanze oder einzelner Teile von ihr oder des Saatgutes oder auch des Kulturbodens. Die Wirkstoffe sind besonders wirksam gegen den Weizensteinbrand (Tilletia caries) und gegen die Streifenkrankheit der Gerste (Helminthosporium gramineum bzw. Drechslera graminea), ferner gegen den Apfelmehltau (Podosphaera leucotricha).

Die gute bakterizide Wirkung besteht insbesondere in einer hohen Aktivität gegen Xanthomonas-Arten.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlenoxide; als feste Trägerstoffe: natürliche Gesteinsmehle, wie Kaoline, Tonerden, Talkum, Kreide, Quarz, Attapulgit, Montmorrillonit oder Diatomeenerde und synthetische Gesteinsmehle, wie hochdisperse Kieselsäure, Aluminiumoxid und Silikate; als feste Trägerstoffe für Granulate: gebrochene und fraktionierte natürliche Gesteine wie Calcit, Marmor, Bims, Sepiolith, Dolomit sowie synthetische Granulate aus anorganischen und organischen Mehlen sowie Granulate aus organischen Material wie Sägemehle, Kokosnußschalen, Maiskolben und Tabakstengel; als Emulgier- und/oder schaumerzeugende Mittel: nichtionogene und anionische Emulgatoren, wie Polyoxyäthylen-Fettsäure-Ester, Polyoxyäthylen-Fettalkohol-Äther, z.B. Alkylaryl-polyglykol-äther, Alkylsulfonate, Alkylsulfate, Arylsulfonate sowie Eiweißhydrolysate; als Dispergiermittel: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Fisenoxid, Titanoxid, Ferrocyanblau und organische Farbstoffe, wie Alizarin-, Azo-Metallphthalocyaninfarbstoffe und Spurennährstoffe wie Salze von Eisen, Mangan, Bor, Kupfer, Kobalt, Molybdän und Zink verwendet werden.

Die Formulierungen enthalten in allgemeinen zwischen 0,1 und 95 Gewichtsprozent Wirkstoff, vorzugsweise zwischen 0,5 und 90%.

Die Wirkstoffe können in den Formulierungen in Mischung mit anderen bekannten Wirkstoffen vorliegen, wie Fungiziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendete werden. Die Anwendung geschieht in üblicher Weise, z.B. durch Gießen, Spritzen, Sprühen, Stäuben, Trockenbeizen, Feuchtbeizen, Naßbeizen, Schlämmbeizen oder Inkrustieren.

Bei der Verwendung als Blattfungizide und Blattbakterizide können die Wirkstoffkonzentrationen in den Anwendungsformen in einem größeren Bereich variiert werden. Sie liegen im allgemeinen zwischen 0,5 und 0,0005 Gew.-%, vorzugsweise zwischen 0,2 und 0,001%.

Bei der Saatgutbehandlung werden im allgemeinen Wirkstoffmengen von 0,01 bis 50 g je Kilogramm Saatgut, vorzugsweise 0,5 bis 5 g, benötigt.

Zur Behandlung sind Wirkstoffmengen von 1 bis 1000 g je cbm Boden, vorzugsweise von 10 bis 200 g, erforderlich.

Die Verwendungsmöglichkeiten gehen aus den nachfolgenden Beispielen hervor:

## Beispiel A

Agarplattentest

Verwendeter Nährboden:

20 Gewichtsteile Agar-Agar
200 Gewichtsteile Kartoffeldekokt
5 Gewichtsteile Malz
15 Gewichtsteile Dextrose
5 Gewichtsteile Pepton
2 Gewichtsteile Dinatriumhydrogenphosphat
0,3 Gewichtsteile Dicalciumnitrat

Verhältnis von Lösungsmittelgemisch zum Nährboden:

2 Gewichtsteile Lösungsmittelgemisch
100 Gewichtsteile Agarnährboden

Zusammensetzung Lösungsmittelgemisch

0,19 Gewichtsteile Dimethylformamid oder Aceton
0,01 Gewichtsteile Emulgator Alkylarylpolyglykoläther
*1,80 Gewichtsteile Wasser*
2 Gewichtsteile Lösungsmittelgemisch

Man vermischt die für die gewünschte Wirkstoffkonzentration im Nährboden nötige Wirkstoffmenge mit der angegebenen Menge des Lösungsmittelgemisches. Das Konzentrat wird im genannten Mengenverhältnis mit dem flüssigen, auf 42°C abgekühlten Nährboden gründlich vermischt und in Petrischalen mit einem Durchmesser von 9 cm gegossen.

Ferner werden Kontrollplatten ohne Präparatbeimischung aufgestellt.

Ist der Nährboden erkaltet und fest, werden die Platten mit den zu prüfenden Pilz- bzw. Bakterienarten beimpft und bei etwa 21°C inkubiert.

Zur Prüfung wurden die Pilzarten Sclerotinia sclerotiorum, Rhizoctonia solani, Pythium ultinum, Cochliobolus miyabeanus, Pyricularia oryzae, Helminthosporium gramineum, pellicularia sasakii und das Bakterium Xanthomonas oryzae herangezogen.

Die Auswertung erfolgt je nach der Wachstumsgeschwindigkeit der Organismen nach 4 bis 10 Tagen. Bei der Auswertung wird das radiale Wachstum auf den behandelten Nährboden mit dem Wachstum auf dem Kontrollnährboden verglichen. Die Bonitierung des Wachstums geschieht mit folgenden Kennzahlen:

1 kein Wachstum
bis 3 sehr starke Hemmung des Wachstums
bis 5 mittelstarke Hemmung des Wachstums
bis 7 schwache Hemmung des Wachstums
9 Wachstum gleich der unbehandelten Kontrolle

Bei diesem Test zeigen z.B. die folgenden bei den Herstellungsbeispielen angegebenen Verbindungen eine überlegene Wirkung gegenüber dem Stand der Technik:
Verbindungen Nr. 1, 27, 20, 9, 37, 38, 10, 16, 23, 22, 2, 24, 11, 17, 5.

Beispiel B

Bakterien-Test/Xanthomonas oryzae

| | | |
|---|---|---|
| Lösungsmittel | 11,75 | Gewichtsteile Aceton |
| Dispergiermittel: | 0,75 | Gewichtsteile Alkylarylglykoläther |
| Wasser | 987,50 | Gewichtsteile |
| andere Zusätze | — | Gewichtsteile — |

Man vermischt die für die gewünschte Wirkstoffkonzentration in der Spritzflüssigkeit nötige Wirkstoffmenge mit der angegebenen Menge des Lösungsmittels und des Dispergiermittels und verdünnt das Konzentrat mit der angegebenen Menge Wasser.

Mit der Spritzflüssigkeit bespritzt man etwa 40 Tage alte Reispflanzen bis zur Tropfnässe. Die Pflanzen verbleiben bis zum Abtrocknen in einem Gewächshaus bei Temperaturen von 22 bis 24°C und einer relativen Luftfeuchtigkeit von etwa 70%. Danach werden Nadeln in eine wässrige Bakteriensuspension von Xanthomonas oryzae getaucht und die Pflanzen durch Anstechen der Blätter inokuliert. Die Pflanzen stehen nach der Inokulation 24 Stunden bei 100% relativer Luftfeuchtigkeit und danach in einem Raum bei 26 bis 28°C und 80% relativer Luftfeuchtigkeit. 10 Tage nach der Inokulation wird der Befall bei allen durch Stich verletzten, inokulierten Blättern von vorher mit Präparat behandelten Pflanzen in Wertzahlen von 1 bis 9 ausgewertet.

1 bedeutet 100%ige Wirkung, 3 = gute Wirkung, 5 = mäßige Wirkung und 9 = keine Wirkung.

Bei diesem Test zeigen z.B. die folgenden bei den Herstellungsbeispielen angegebenen Verbindungen eine überlegene Wirkung gegenüber dem Stand der Technik:
Verbindungen Nr. 12, 15, 1, 43, 36, 35.

Beispiel C

Saatgutbeizmittel-Test/Weizensteinbrand (samenbürtige Mykose)

Zur Herstellung eines zweckmäßigen Trockenbeizmittels verstreckt man den Wirkstoff mit einem Gemisch aus gleichen Gewichtsteilen Talkum und Kieselgur zu einer feinpulverigen Mischung mit der gewünschten Wirkstoffkonzentration.

Man kontaminiert Weizensaatgut mit 5 g Chlamydosporen von Tilletia caries pro kg Saatgut. Zur Beizung schüttelt man das Saatgut mit dem Beizmittel in einer verschlossenen Glasflasche. Das Saatgut wird auf feuchtem Lehm unter einer Dreckschicht aus einer Lage Mull und 2 cm mäßig feuchter Komposterde 10 Tage lang im Kühlschrank bei 10°C optimalen Keimungsbedingungen für die Sporen ausgesetzt.

Anschließend bestimmt man mikroskopisch die Keimung der Sporen auf den Weizenkörner, die jeweils mit rund 100 000 Sporen besetzt sind. Der Wirkstoff ist umso wirksamer, je weniger Sporen gekeimt sind.

Bei diesem Test zeigen z.B. die folgenden bei den Herstellungsbeispielen angegebenen Verbindungen eine überlegene Wirkung gegenüber dem Stand der Technik:
Verbindungen Nr. 1, 43, 36, 25, 26, 27, 20, 9, 37, 38, 10, 23, 21, 22, 2, 24, 11, 17. 6, 31, 18, 19.

Beispiel D

Saatgutbeizmittel-Test/Streifenkrankheit der Gerste (samenbürtige Mykose)

Zur Herstellung eines zweckmäßigen Trockenbeizmittels verstreckt man den Wirkstoff mit einem Gemisch aus gleichen Gewichtsteilen Talkum und Kieselgur zu einer feinpulverigen Mischung mit der gewünschten Wirkstoffkonzentration.

Zur Beizung schüttelt man Gerstensaatgut, das durch Drechslera graminea (Synonym Helminthosporium gramineum) natürlich verseucht ist, mit dem Beizmittel in einer verschlossenen Glasflasche. Das Saatgut setzt man auf feuchten Filterscheiben in verschlossenen Petrischalen im Kühlschrank 10 Tage lang einer Temperatur von 4°C aus. Dabei wird die Keimung der Gerste und gegebenenfalls auch der Pilzsporen eingeleitet. Anschließend sät man die vorgekeimte Gerste mit 2 x 50 Korn 2 cm tief in Fruhstorfer Einheitserde und kultiviert sie im Gewächshaus bei Temperaturen um 18°C in Saatkästen, die täglich 16 Stunden dem Licht ausgesetzt werden. Innerhalb von 3 bis 4 Wochen bilden sich die typischen Symptome der Streifenkrankheit aus.

Nach dieser Zeit bestimmt man die Anzahl der kranken Pflanzen in Prozent der insgesamt aufgelaufenen Pflanzen. Der Wirkstoff ist umso wirksamer, je weniger Pflanzen erkrankt sind.

Bei diesem Test zeigen z.B. die folgenden bei den Herstellungsbeispielen angegebenen Verbindungen eine überlegene Wirkung gegenüber dem Stand der Technik:
Verbindungen Nr. 1, 26, 5, 10, 16, 23, 2.

Herstellungsbeispiele

Beispiel 1
(Verfahren a))

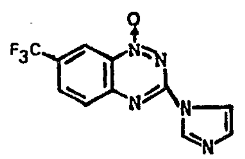

Zur Lösung von 25 g (0,1 Mol) 3-Chlor-7-trifluormethyl-benzo-1,2,4-triazin-1-oxid in 100 ml Dioxan tropft man bei Raumtemperatur eine Mischung aus 7 g (0,103 Mol) Imidazol, 10,1 g (0,1 Mol) Triäthylamin und 50 ml Dioxan. Das Reaktionsgemisch wird allmählich zum Sieden erhitzt und 5 Stunden unter Rückfluß gekocht. Nach dem Abkühlen versetzt man mit dem dreifachen Volumen Wasser, saugt das Reaktionsprodukt ab und trocknet es. Man erhält 17 g 3-Imidazolyl-(1)-7-trifluormethyl-benzo-1,2,4-triazin-1-oxid vom Fp. 128°C (aus Waschbenzin ungelöst), das sind 60,5% der Theorie.

Beispiel 2
(Verfahren a))

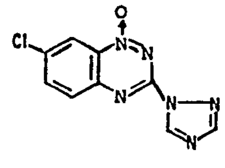

In die Lösung von 6,9 g (0,1 Mol) Triazol in 50 ml Pyridin werden bei Raumtemperatur unter Rühren portionsweise 21,6 g (0,1 Mol) 3,7-Dichlor-benzo-1,2,4-triazin-1-oxid eingetragen. Das Gemisch wird 1 Stunde bei Raumtemperatur gerührt und dann 3 Stunden unter Rückfluß gekocht. Das Pyridin wird im Vakuum abdestilliert, der Rückstand mit stark verdünnter Salzsäure verrührt, abgesaugt, mit verdünnter Salzsäure und Wasser gewaschen und getrocknet. Man erhält 18 g 3-(1,2,4-Triazol-1-yl)-7-chlor-benzo-1,2,4-triazin-1-oxid vom Fp. 204—205°C (aus Butanol ungelöst), das sind 72,6% der Theorie.

Beispiel 3
(Verfahren a))

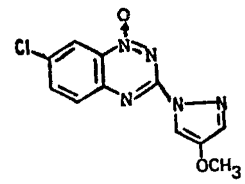

In die Lösung von 12,6 g (0,13 Mol) 4-Methoxy-pyrazol in 100 ml absol. Dimethylformamid trägt man bei Raumtemperatur 3,45 g (0,115 Mol) Natriumhydrid (80%ig) ein und erwärmt kurz auf 60°C nach. Die auf Raumtemperatur gekühlte Mischung wird portionsweise mit 21,6 g (0,1 Mol) 3,7-Dichlor-benzo-1,2,4-triazin-1-oxid versetzt. Nach Abklingen der exothermen Reaktion wird noch während 3 Stunden bei 30 bis 40°C nachgerührt und das Reaktionsprodukt anschließend mit Wasser ausgefällt. Dieses wird abgesaugt, in 150 ml Methanol verrührt, erneut abgesaugt und getrocknet. Man erhält 17,3 g 3-(4-Methoxy-pyrazol-1-yl)-7-chlor-benzo-1,2,4-triazin-1-oxid vom Fp. 253—255°C (unter Zersetzung), das sind 62,3% der Theorie. Die Verbindung läßt sich aus Dimethylformamid umlösen.

In entsprechender Weise werden die folgenden Verbindungen der allgemeinen Formel

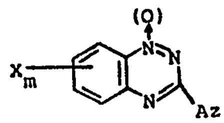

erhalten:

| Beispiel Nr. | $X_m$ | Az | Fp (°C) |
|---|---|---|---|
| 4 | 7-Cl | 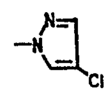 | 268–270 (Zers.) |
| 5 | 7-Cl | 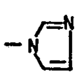 | 172 |
| 6 | 6-Cl, 7-Cl | 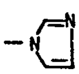 | 200 |
| 7 | 5-Cl, 7-Cl | 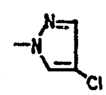 | 210–211 |
| 8 | 5-Cl, 7-Cl | 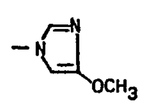 | 224–226 |
| 9 | 5-Cl, 7-Cl | 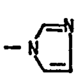 | 144 |
| 10 | 5-Cl, 7-Cl | 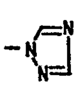 | 206 |
| 11 | 7-Br | 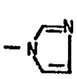 | 160 |

Beispiel 12
(Reduktion)

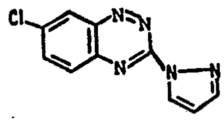

24,8 g (0,1 Mol) 3-Pyrazol-1-yl-7-chlor-benzo-1,2,4-triazin-1-oxid werden in 150 ml Aethanol bei 25 bis 30°C in Gegenwart von Raney-Nickel unter einem Wasserstoffdruck von 50 bar bis zur Druckkonstanz hydriert. Der Katalysator wird abfiltriert und die Lösung im Vakuum eingedampft. Man erhält in praktisch quantitativer Ausbeute 3-Pyrazol-1-yl-7-chlor-benzo-1,2,4-triazin vom Fp. 188°C. Die Verbindung läßt sich aus Toluol umlösen.

In entsprechender Weise werden die folgenden Verbindungen der allgemeinen Formel

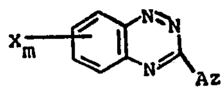

erhalten:

| Beispiel Nr. | $X_m$ | Az | Fp (°C) |
|---|---|---|---|
| 13 | 6-Cl | 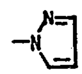 | 110 |
| 14 | 7-Cl | 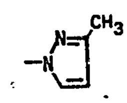 | 168 |
| 15 | 7-Cl | 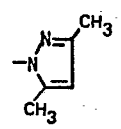 | 174 |
| 16 | 7-Cl | 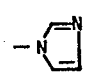 | 158 |
| 17 | 7-Cl | 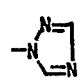 | 194 |
| 18 | 7-Cl | 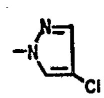 | 228,5–229,5 |
| 19 | 7-Cl | 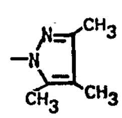 | 194–195 |
| 20 | 5-Cl, 7-Cl | 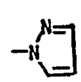 | >250 |

| Beispiel Nr. | $X_m$ | Az | Fp (°C) |
|---|---|---|---|
| 21 | 5-Cl, 7-Cl | 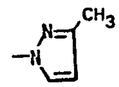 | 176 |
| 22 | 5-Cl, 7-Cl | 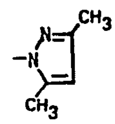 | 124 |
| 23 | 5-Cl, 7-Cl | 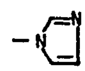 | 184 |
| 24 | 5-Cl, 7-Cl | 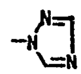 | 150 |
| 25 | 7-$CF_3$ | 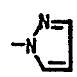 | 210 |
| 26 | 7-$CF_3$ | 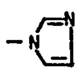 | 130 |
| 27 | 7-$OCH_3$ | 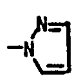 | 174 |

Beispiel 15a
(Verfahren b), andere Herst.als Beispiel 15)

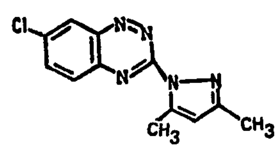

19,5 (0,1 Mol) 3-Hydrazino-7-chlor-benzo-1,2,4-triazin und 10 g (0,1 Mol) Acetylaceton werden in 125 ml Aethanol 5 Stunden unter Rückfluß gekocht. Der nach Abdestillieren des Lösungsmittels verbleibende Rückstand wird aus Waschbenzin umkristallisiert. Man erhält 22,3 g 3-(3,5-Dimethyl-pyrazol-1-yl)-7-chlor-benzo-1,2,4-triazin vom Fp. 174°C, das sind 86% der Theorie.

Beispiel 28
(Verfahren b))

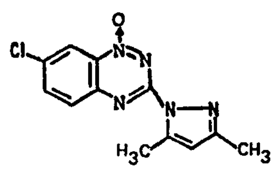

21,2 g (0,1 Mol) 7-Chlor-3-hydrazino-benzo-1,2,4-triazin-1-oxid werden mit 10 g (0,1 Mol) Acetylaceton in 200 ml Aethanol 5 Stunden unter Rückfluß gekocht. Nach dem Abkühlen wird das ausgefallene Reaktionsprodukt abgesaugt, mit Aethanol gewaschen und getrocknet. Man erhält 20,2 g (3,5-Dimethyl-pyrazol-1-yl)-7-chlor-benzo-1,2,4-triazin-1-oxid vom Fp. 198—200° (aus Dimethylformamid), das sind 73% der Theorie.

Vorprodukte

3-Hydrazino-benzo-1,2,4-triazin-1-oxide (Vorstufe A)

In eine Mischung aus 200 g (4 Mol) Hydrazinhydrat und 1,2 ltr. Dioxan trägt man bei Raumtemperatur unter Rühren innerhalb 1 Stunde portionsweise 1 Mol eines 3-Chlor-benzo-1,2,4-triazin-1-oxids ein. Nach Abklingen der schwach exothermen Reaktion rührt man das Gemisch noch 3 Stunden bei 50 bis 60°C nach. Nach Abkühlen auf 15 bis 20°C wird das ausgefallene Reaktionsprodukt abgesaugt, mit wenig Dioxan, dann mit Wasser gewaschen und getrocknet. Eine weitere Menge des Reaktionsproduktes kann durch Einengen der Dioxanlösung oder (weniger rein) durch Ausfällen mit Wasser gewonnen werden.

Auf diese Weise erhält man in Ausbeuten über 85% die folgenden 3-Hydrazino-benzo-1,2,4-triazin-1-oxide:

| | |
|---|---|
| 6-Chlor-3-hydrazino-benzotriazin-1-oxid | Fp. 178—180°C |
| 7-Chlor-3-hydrazino-benzotriazin-1-oxid | Fp. 198—200°C (aus Dimethylformamid) |
| 5,7-Dichlor-3-hydrazino-benzotriazin-1-oxid | Fp. 210°C |
| 6,7-Dichlor-3-hydrazino-benzotriazin-1-oxid | Fp. 205°C (aus Glykolmonomethyläther) |
| 7-Brom-3-hydrazino-benzotriazin-1-oxid | Fp. 217°C |
| 7-Trifluormethyl-3-hydrazino-benzotriazin-1-oxid | Fp. 164—166°C |
| 7-Methoxy-3-hydrazino-benzotriazin-1-oxid | Fp. 170—172°C |

3-Hydrazino-benzo-1,2,4-triazine (Vorstufe B)

In die Mischung aus 17,1 g (0,3 Mol) Natriumhydrogensulfid in 80 ml Wasser und 250 ml Dioxan trägt man bei Raumtemperatur unter Rühren portionsweise 0,1 Mol eines 3-Hydrazino-benzo-1,2,4-triazin-1-oxids (Vorstufe A) ein. Das Gemisch wird 1 Stunde bei Raumtemperatur und noch 3 Stunden bei 50°C nachgerührt. Dann destilliert man das Dioxan in Vakuum ab und verrührt den Rückstand mit 200 ml Wasser. Das dabei unlösliche Reaktionsprodukt wird abgesaugt, mit Wasser gewaschen und getrocknet. Kleine Mengen an gleichzeitig ausgefallenem Schwefel können durch Waschen mit Schwefelkohlenstoff entfernt werden.

Auf diese Weise erhält man z.B.:

| | |
|---|---|
| 7-Chlor-3-hydrazino-benzo-1,2,4-triazin | Fp. 190—192°C (aus Dimethylformamid) in 72% Ausbeute |
| 5,7-Dichlor-hydrazino-benzo-1,2,4-triazin | Fp. 236—238°C (Butanol) in 67% Ausbeute |

Beispiel 29
(Verfahren b))

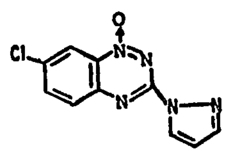

21,2 g (0,1 Mol) 7-Chlor-3-hydrazino-benzo-1,2,4-triazin-1-oxid werden mit 20 g (0,12 Mol) 1,1,3,3-Tetramethoxypropan in 160 ml Aethanol unter Zusatz von 10 ml konz. Salzsäure während 5 Stunden unter Rückfluß erhitzt. Man kühlt dann auf Raumtemperatur ab, saugt das Reaktionsprodukt ab, wäscht et mit Aethanol und trocknet. Man erhält 18 g 3-Pyrazol-1-yl-7-chlor-benzo-1,2,4-triazin-1-oxid vom Fp. 210°C (aus Butanol umgelöst).

Beispiel 30
(Verfahren b))

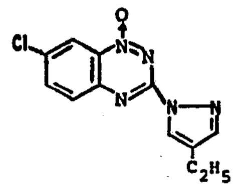

Zur Mischung aus 21,15 g (0,1 Mol) 3-Hydrazino-7-chlor-benzo-1,2,4-triazin-1-oxid, 30 ml Methanol und 125 ml (0,25 Mol) 2n-Salzsäure gibt man bei 60°C 15,24 g (0,12 Mol) 2-Aethyl-3-dimethylamino-acrolein, wobei die Temperatur auf 68°C ansteigt. Das Gemisch wird während 8 Stunden bei 60°C gerührt, auf Raumtemperatur abgekühlt und abgesaugt. Der Filterkuchen wird mit Wasser und Methanol gewaschen und bei 110°C getrocknet. Man erhält 13 g 3-(4-Aethyl-pyrazol-1-yl)-7-chlor-benzo-1,2,4-triazin-1-oxid vom Fp. 228,5 bis 230° (aus Toluol umgelöst), das sind 47,2% der Theorie.

In entsprechender Weise werden die Verbindungen der allgemeinen Formel

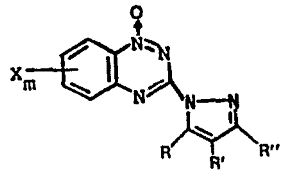

erhalten:

| Beispiel Nr. | Reaktionskomponente | $X_m$ | R | R′ | R″ | Fp (°C) |
|---|---|---|---|---|---|---|
| 31 | 1,1,3,3-Tetramethoxypropan | 6-Cl | H | H | H | 200 |
| 32 | 4,4-Dimethoxy-butanon-(2) | 7-Cl | H | H | $CH_3$ | 112 |
| 33 | Äthoxymethylen-cyanessigsäure-äthylester | 7-Cl | $NH_2$ | $CO-OC_2H_5$ | H | 269–274 (unter Zers.) |
| 34 | 3-Methyl-pentandion-(2,4) | 7-Cl | $CH_3$ | $CH_3$ | $CH_3$ | 228–231 |
| 35 | 2-Isopropoxy-3-dimethylamino-acrolein | 7-Cl | H | $OCH(CH_3)_2$ | H | 195–196 |
| 36 | 1,1,3,3-Tetramethoxypropan | 5-Cl, 7-Cl | H | H | H | 160 |
| 37 | 4,4-Dimethoxy-butanon-(2) | 5-Cl, 7-Cl | H | H | $CH_3$ | 170 |
| 38 | Acetylaceton | 5-Cl, 7-Cl | $CH_3$ | H | $CH_3$ | 174 |
| 39 | 3-Methyl-pentandion-(2,4) | 5-Cl, 7-Cl | $CH_3$ | $CH_3$ | $CH_3$ | 198–202 |
| 40 | 2-Aethyl-3-dimethylamino-acrolein | 5-Cl, 7-Cl | H | $C_2H_5$ | H | |
| 41 | 1,1,3,3-Tetramethoxypropan | 7-Br | H | H | H | 220 |
| 42 | 1,1,3,3-Tetramethoxypropan | $7-CF_3$ | H | H | H | 230 |
| 43 | 1,1,3,3-Tetramethoxypropan | $7-OCH_3$ | H | H | H | 168 |

**Patentansprüche**

1. Fungizide und bakterizide Mittel im Pflanzenschutz, gekennzeichnet durch einem Gehalt an mindestens einem 3-Azolyl-benzo-1,2,4-triazin oder -benzo-1,2,4-triazin-1-oxid der allgemeinen Formel

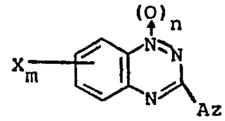

(I)

in welcher

X für Halogen, Alkyl-, Alkoxy-, Alkylmercapto- oder Alkylsulfonyl-Gruppen mit jeweils bis zu 4 Kohlenstoffatomen, oder für eine Nitro- oder Trifluormethyl-Gruppe steht,

m für ganze Zahlen von 0 bis 4 steht,

n für die Zahlen 0 oder 1 steht und

Az für einen über Stickstoff gebundenen fünfgliedrigen hetero-aromatischen Ring mit 2 oder 3 Stickstoffatomen steht, der gegebenenfalls substituiert sein kann durch Alkyl, Alkoxy oder Alkylmercapto mit jeweils 1 bis 6 Kohlenstoffatomen, durch Halogen, Hydroxy, durch Aryl- und Aralkyl-Reste, ferner durch Amino-, Mono- oder Dialkylamino-Gruppen mit 1 bis 2 Kohlenstoffatomen in den jeweiligen Alkylgruppen, durch Alkoxyalkoxy-, Acylamino-, Carboxyl-, Carbonsäureester- und gegebenenfalls N-substituierte Carbonamido-Gruppen und durch die Cyano-Gruppe.

2. Verwendung von 3-Azolyl-benzo-1,2,4-triazinen oder -benzo-2,4-triazin-1-oxiden gemäß Anspruch 1 zur Bekämpfung von Pilzen und Bakterien im Pflanzenschutz.

**Revendications**

1. Compositions fongicides et bactéricides pour la protection des plantes, caractérisées par une teneur en au moins une 3-azolylbenzo-1,2,4-triazine ou en un 1-oxyde de 3-azolylbenzo-1,2,4-triazine de formule générale:

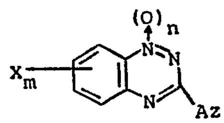

(I)

dans laquelle

X représente un halogène, des groupes alkyle, alkoxy, alkylmercapto ou alkylsulfonyle ayant chacun jusqu'à 4 atomes de carbone ou un groupe nitro ou trifluorométhyle,

*m* représente les nombres entiers de 0 à 4,

*n* représente les nombres 0 ou 1 et

Az désigne un noyau hétéro-aromatique pentagonal attaché par un atome d'azote et ayant 2 ou 3 atomes d'azote, qui peut être substitué le cas échéant par des restes alkyle, alkoxy ou alkylmercapto ayant chacun 1 à 6 atomes de carbone, par un halogène ou par des restes hydroxy, aryle ou aralkyle,

ainsi que par des groupes amino, monoalkylamino ou dialkylamino dont chaque radical alkyle comprend 1 ou 2 atomes de carbone, par des groupes alkoxyalkoxy, acylamino, carboxyle, ester d'acide carboxylique et le cas échéant carbonamido substitué sur l'atome d'azote et par le groupe cyano.

2. Utilisation de 3-azolylbenzo-1,2,4-triazines ou 1-oxydes de 3-azolylbenzo-2,4-triazines suivant la revendication 1 pour la lutte contre des champignons et des bactéries dans la protection des plantes.

**Claims**

1. Fungicidal and bactericidal agents in plant protection, characterised in that they contain at least one 3-azolyl-benzo-1,2,4-triazine or -benzo-1,2,4-triazine 1-oxide of the general formula

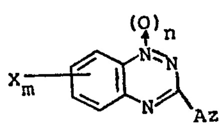

(I)

in which

X represents halogen, alkyl, alkoxy, alkylmercapto or alkylsulphonyl groups with in each case up to 4 carbon atoms, or a nitro or trifluoromethyl group,

m represents an integer from 0 to 4,

n represents the number 0 or 1 and

Az represents a five-membered hetero-aromatic ring which has 2 or 3 nitrogen atoms, is bonded via nitrogen and can optionally be substituted by alkyl, alkoxy or alkylmercapto with in each case 1 to 6 carbon atoms, by halogen or hydroxyl, by aryl or aralkyl radicals, and furthermore by amino or mono- or di-alkylamino groups with 1 to 2 carbon atoms in the particular alkyl groups, by alkoxyalkoxy, acylamino, carboxyl, carboxylic acid ester or optionally N-substituted carboxamido groups or by the cyano group.

2. Use of 3-azolyl-benzo-1,2,4-triazines or -benzo-2,4-triazine 1-oxides according to Claim 1 for combating fungi and bacteria in plant protection.